# EUROPEAN PATENT SPECIFICATION

(11) **EP 4 272 657 B1**
(45) Date of publication and mention of the grant of the patent: **28.01.2026**
(21) Application number: 22742528.7
(22) Date of filing: 17.01.2022
(51) Int. Cl.: A61B 17/00, A61M 37/00

(54) **OPERATING DEVICE AND IMPLANT INDWELLING TOOL**
BETRIEBSVORRICHTUNG UND IMPLANTATVERWEILWERKZEUG
DISPOSITIF D'ACTIONNEMENT ET OUTIL D'IMPLANT À DEMEURE

(30) Priority: 19.01.2021 JP 2021006142
(43) Date of publication of application: 08.11.2023
(73) Proprietor: TERUMO Kabushiki Kaisha, Tokyo 151-0072 (JP)
(72) Inventor: ISHIDA, Masahiro, Ashigarakami-gun, Kanagawa 259-0151 (JP)
(74) Representative: Casalonga
(86) International application number: PCT/JP2022/001261
(87) International publication number: WO 2022/158406

(56) References cited:
- WO-A1-2017/163600
- WO-A1-2020/189157
- WO-A2-2020/039314
- JP-A- 2016 513 575
- JP-A- 2016 513 575
- JP-A- 2020 525 160
- JP-B2- 6 737 870

## Description

### Technical Field

The present invention relates to an operation device and an implanted body indwelling instrument for indwelling an implanted body in a body.

### Background Art

Various therapeutic methods for indwelling an implanted body in a living tissue have been proposed. For example, JP 2020-127607 A describes a device in which a porous collagen fiber having an infinite number of pores formed therein is implanted in a damaged site to promote regeneration of the damaged tissue.
Patent document JP 6 737870 B2 discloses a medical bidirectional suture device capable of suturing an object with a filament in both directions from the proximal side to the distal side and from the distal side to the proximal side, and a method for operating the same.
Document JP 2016 513575 A discloses an instrument capable of ensuring that a practitioner can pass a suture from a remote access point into the subcutaneous tissue and a surgical method facilitated by such an instrument.

### Summary of Invention

Claim 1 defines the invention and dependent claims disclose embodiments. No surgical methods are claimed per-se. In order to reliably indwell a thread-like implanted body in a biological tissue, it is desirable to reliably hold and release one end of the implanted body. It is therefore conceivable to use a suture device for introducing a suture thread as described in U.S. Patent No. 9301748 B. This device has a structure in which a tweezer-like gripping member is opened and closed with a wire.

However, in the device described in U.S. Patent No. 9301748 B, it is necessary to greatly expand a distal end in a radial direction when the gripping member is released, and there is a possibility that the device does not reliably operate inside the living tissue subject to pressure.

Thus, an object of one embodiment is to provide an operation device and an implanted body indwelling instrument capable of reliably indwelling an implanted body in a body.

One aspect of the following disclosure is an operation device including: an outer cylinder in which a lumen penetrating in an axial direction is formed; a shaft disposed in the lumen; and a gripping mechanism that is provided in the outer cylinder and the shaft and grips a thread-like implanted body in a switchable manner between a fixed state in which the thread-like implanted body is undetachable and a released state in which the thread-like implanted body is detachable, in which the gripping mechanism includes: a first insertion hole penetrating a side portion of the outer cylinder in a radial direction; a second insertion hole provided in the shaft at a portion corresponding to the first insertion hole and penetrating a side portion of the shaft in the radial direction; and a rotation mechanism that rotates the shaft relative to the outer cylinder, and the implanted body is inserted into the first insertion hole and the second insertion hole and gripped.

Another aspect is an implanted body indwelling instrument including: the operation device according to the above aspect; and a puncture needle having an outer needle into which the outer cylinder of the operation device can be inserted in a state where the implanted body is gripped.

According to the operation device and the implanted body indwelling instrument from the above viewpoint, the implanted body can be reliably indwelled in the body.

### Brief Description of Drawings

Fig. 1 is a perspective view of an implanted body indwelling instrument according to an embodiment in a separated state.
Fig. 2 is a perspective view illustrating components of an operation device of Fig. 1.
Fig. 3A is an enlarged perspective view of a distal end of an outer cylinder illustrated in Fig. 2, and Fig. 3B is a side view of the distal end of Fig. 3A.
Fig. 4A is a perspective view of an outer cylinder hub of Fig. 2, and Fig. 4B is a cross-sectional view of the outer cylinder hub taken along a line IVB-IVB of Fig. 4A.
Fig. 5A is an enlarged perspective view of a distal end of a shaft in Fig. 2, and Fig. 5B is a side view of the distal end in Fig. 5A.
Fig. 6 is a perspective view of a shaft hub of Fig. 2.
Fig. 7 is an enlarged cross-sectional view of the vicinity of an operation portion of the shaft hub in a released state.
Fig. 8 is an exploded perspective view illustrating a rotation mechanism of the shaft hub of Fig. 2.
Fig. 9A is a cross-sectional view of a state in which the operation portion of the shaft hub is pulled out from a rotation shaft portion, and Fig. 9B is a cross-sectional view of a state in which the operation portion of the shaft hub is pushed into the rotation shaft portion.
Fig. 10 is a perspective cross-sectional view of the outer cylinder hub and the shaft hub of the operation device of Fig. 1.
Fig. 11 is a cutout perspective view illustrating an internal structure of the outer cylinder hub of Fig. 1, in which part of the outer cylinder hub is cut out.
Fig. 12 is a cross-sectional view illustrating the outer cylinder hub of Fig. 1, in which an upper portion is cut out.
Fig. 13 is a perspective view illustrating components constituting a puncture needle of Fig. 1.
Fig. 14A is a perspective view of the outer cylinder and the shaft in a released state, and Fig. 14B is a cross-sectional view illustrating operation of the shaft hub in the released state of Fig. 14A.
Fig. 15A is a perspective view of the outer cylinder and the shaft in a fixed state, and Fig. 15B is a cross-sectional view illustrating operation of the shaft hub in the fixed state of Fig. 15A.
Fig. 16 is an explanatory view illustrating a state in which a skin of a living body is punctured with a puncture needle.
Fig. 17 is an explanatory view illustrating operation after an inner needle assembly is pulled out from the puncture needle.
Fig. 18 is an explanatory view illustrating operation of inserting an operation device into an outer needle assembly.
Fig. 19A is a top view illustrating operation of pressing the outer cylinder hub of the operation device against the skin, and Fig. 19B is a side view of Fig. 19A.
Fig. 20A is a top view illustrating a positional relationship between a lock projection and an outer needle hub, and Fig. 20B is a side view of Fig. 20A.
Fig. 21A is a top view illustrating a positional relationship between the lock projection and the outer needle hub when a distal end of the operation device is caused to project from an outer needle, and Fig. 21B is a side view of Fig. 21A.
Fig. 22A is a cross-sectional view illustrating operation of putting the operation device into a released state, and Fig. 22B is a perspective view illustrating a released state of the outer cylinder and the shaft of the operation device.
Fig. 23 is an explanatory view illustrating operation of pulling out the outer needle assembly and the operation device from the skin while leaving the implanted body.

### Description of Embodiments

Hereinafter, preferred embodiments of an operation device and an implanted body indwelling instrument will be described in detail with reference to the accompanying drawings. Note that in the following description, a hand side when the device is handled by a user is referred to as a "proximal end side", and a needle tip side away from the user is referred to as a "distal end side".

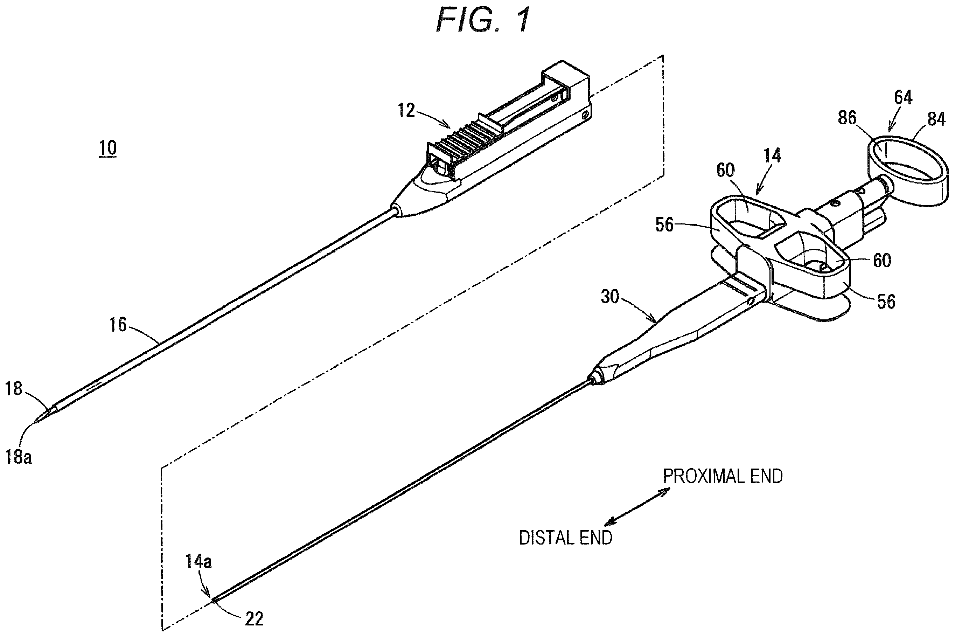

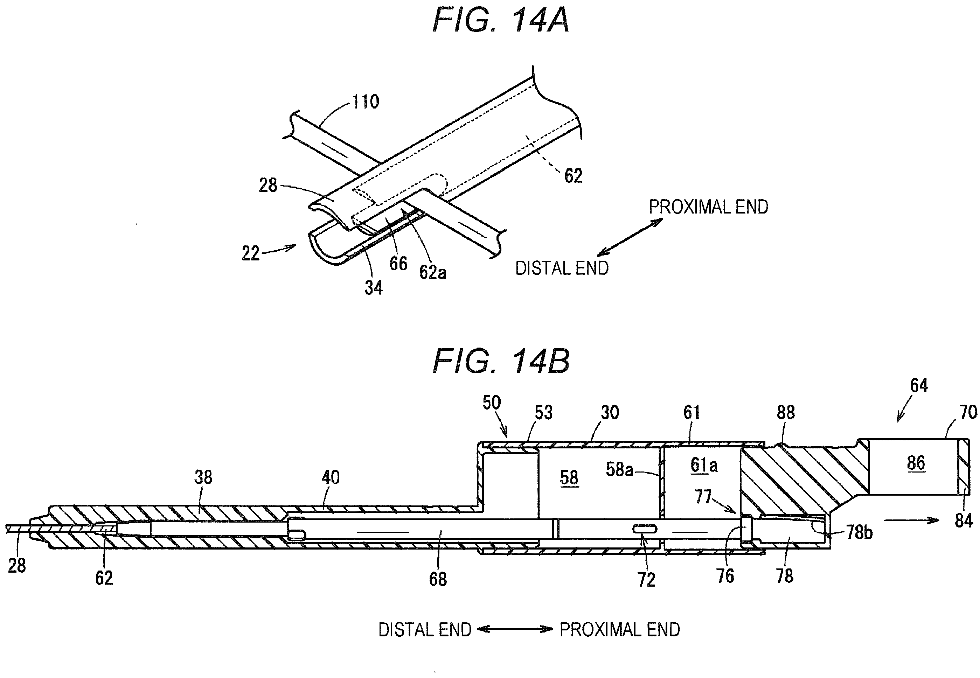

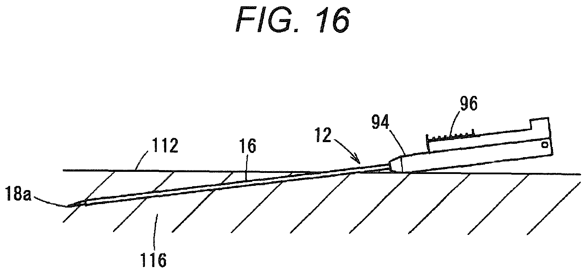

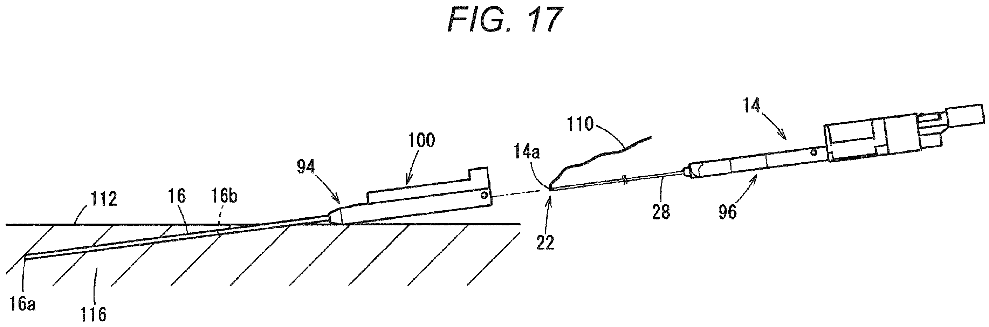

As illustrated in Fig. 1, an implanted body indwelling instrument 10 according to the present embodiment includes a puncture needle 12 and an operation device 14. The puncture needle 12 includes an outer needle 16 and an inner needle 18, a sharp needle tip is formed at a distal end 18a of the inner needle 18, and the outer needle 16 and the inner needle 18 are used by puncturing a target site inside a living tissue 116 from the skin 112 (see Fig. 16). The operation device 14 is used by being inserted into an inner side of the outer needle 16 after puncturing the target site with the puncture needle 12. The operation device 14 includes a gripping mechanism 22 that holds a fibrous implanted body 110 (see Fig. 17, and the like) at a distal end 14a and is inserted into the outer needle 16 of the puncture needle 12 in a state where an implanted body 110 (see Fig. 14A) is gripped at the distal end 14a and conveys the implanted body 110 to the target site.

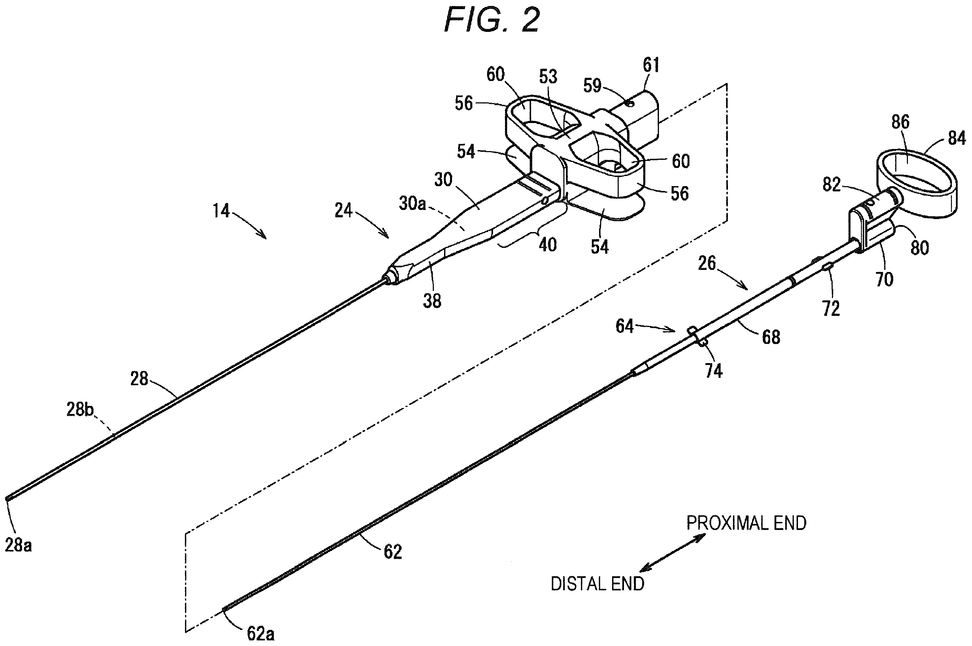

As illustrated in Fig. 2, the operation device 14 includes an outer cylinder assembly 24 and a shaft assembly 26. The operation device 14 is assembled by arranging the shaft assembly 26 inside the outer cylinder assembly 24.

The outer cylinder assembly 24 includes an elongated outer cylinder 28 and an outer cylinder hub 30 joined to a proximal end side of the outer cylinder 28. The outer cylinder 28 is a cylindrical member formed with a metal such as stainless steel, a hard resin, or the like, and is formed inside such that a lumen 28b penetrates in an axial direction. A diameter of the outer cylinder 28 is formed to have an outer diameter dimension capable of being inserted into the outer needle 16 of the puncture needle 12 illustrated in Fig. 1. The lumen 28b of the outer cylinder 28 is open to the proximal end side and communicates with a cavity 30a of the outer cylinder hub 30.

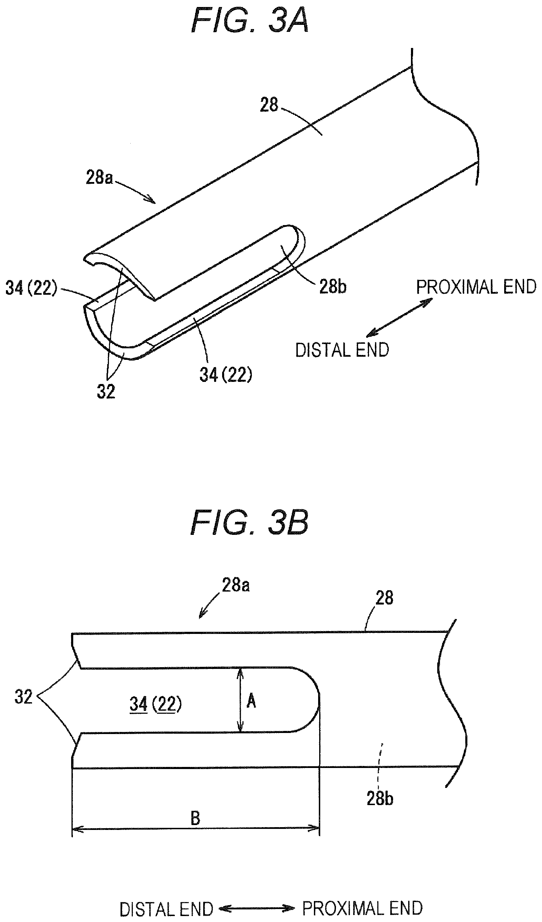

As illustrated in Fig. 3A, an end surface 32 substantially perpendicular to the axial direction of the outer cylinder 28 is formed at a distal end 28a of the outer cylinder 28. A pair of first insertion holes 34 constituting part of the gripping mechanism 22 is formed in an outer peripheral portion closer to the proximal end than the end surface 32. The first insertion hole 34 is disposed so as to face each other across a central axis of the outer cylinder 28. The first insertion hole 34 holds the implanted body 110 so as to penetrate the implanted body in the radial direction of the outer cylinder 28.

The first insertion hole 34 has a circumferential dimension A larger than a diameter of the implanted body 110. In addition, the first insertion hole 34 extends long in the axial direction, and a dimension B in the axial direction is larger than the dimension A in the circumferential direction. The first insertion hole 34 is opened at the end surface 32 and formed in a groove shape. The first insertion hole 34 does not have to be opened to the end surface 32, but is preferably opened to the end surface 32 because work of arranging the thin implanted body 110 can be easily performed.

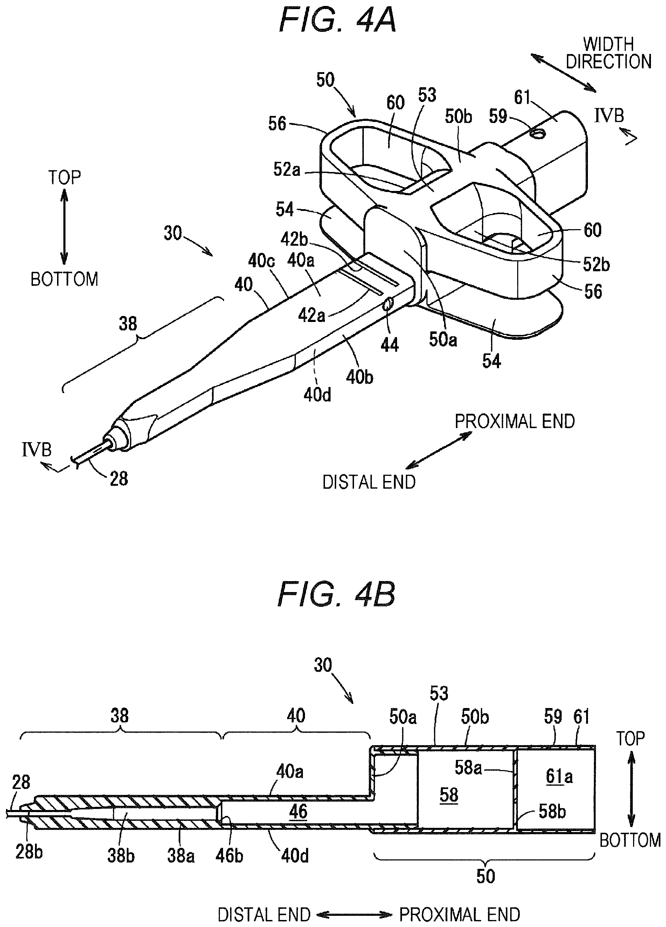

As illustrated in Fig. 4A, the outer cylinder hub 30 is joined to a proximal end of the outer cylinder 28. The outer cylinder hub 30 is formed with, for example, a hard resin material such as a polycarbonate resin. A connection portion 38 for holding the outer cylinder 28 is provided on the distal end side of the outer cylinder hub 30. The connection portion 38 is a portion formed in a tapered shape toward the distal end side, and the inside thereof is joined in close contact with an outer peripheral portion of the outer cylinder 28. A rectangular tube portion 40 having a rectangular cross section is formed on a proximal end side of the connection portion 38.

The rectangular tube portion 40 is a tubular portion having a rectangular cross section perpendicular to the axial direction and is formed to be elongated in the axial direction and thin in the vertical direction. The rectangular tube portion 40 has a flat upper surface 40a at an upper end, side surfaces 40b and 40c on both sides, and a flat bottom surface 40d at a lower end. In the vicinity of a proximal end of the upper surface 40a, a first graduation line 42a and a second graduation line 42b are formed as grooves extending in a width direction. A lock projection 44 is formed on each of the side surfaces 40b and 40c.

As illustrated in Fig. 4B, a first cavity 46 is formed inside the rectangular tube portion 40. The first cavity 46 is formed to extend in the axial direction of the rectangular tube portion 40. A thick portion 38a of the connection portion 38 is formed on a distal end side of the first cavity 46. Inside the thick portion 38a, a shaft hole 38b is formed penetrating in the axial direction. The outer cylinder 28 is inserted and joined to a distal end of the shaft hole 38b, and the distal end of the shaft hole 38b communicates with the lumen 28b of the outer cylinder 28. A distal end wall 46b is formed as a wall portion at the distal end of the first cavity 46 (a proximal end of the thick portion 38a). The shaft hole 38b opens at the distal end wall 46b and communicates with the first cavity 46. A rotation shaft portion 68 (described later) of the shaft assembly 26 is rotatably accommodated in the shaft hole 38b and the first cavity 46.

As illustrated in Fig. 4A, a joint portion 50a for attaching a molded body 50b is formed at a proximal end of the rectangular tube portion 40. The joint portion 50a is formed in a wall shape rising upward from the proximal end of the rectangular tube portion 40. The joint portion 50a constitutes part of the distal end side of a grip portion 50.

The grip portion 50 is provided on a proximal end side of the rectangular tube portion 40. As illustrated in Fig. 4B, the grip portion 50 includes the joint portion 50a and the molded body 50b connected to the proximal end side of the joint portion 50a. The molded body 50b is formed with a resin material similar to that of the rectangular tube portion 40 and is joined to the proximal end side of the joint portion 50a by a method such as adhesion, welding, or caulking.

As illustrated in Fig. 4A, the grip portion 50 includes a main body portion 53, a pair of support plates 54, a pair of grip rings 56, and a housing portion 61. The main body portion 53 is a tubular member formed in a box shape and is formed to have a larger dimension in the vertical direction than the rectangular tube portion 40.

As illustrated in Fig. 4B, a second cavity 58 and a proximal end wall 58a are formed inside the main body portion 53. A distal end side of the second cavity 58 communicates with the first cavity 46 of the rectangular tube portion 40. The proximal end wall 58a is provided on a proximal end side of the second cavity 58. The proximal end wall 58a is provided on the proximal end side of the second cavity 58 and partitions the proximal end side of the second cavity 58. A shaft hole 58b through which the rotation shaft portion 68 (see Fig. 2) is to be inserted is formed in the proximal end wall 58a.

As illustrated in Fig. 4A, the housing portion 61 is formed on a proximal end side of the main body portion 53. The housing portion 61 is a cylindrical portion formed in an oval shape (non-circular shape) long in the vertical direction. As illustrated in Fig. 4B, a housing hole 61a is formed inside the housing portion 61. The housing hole 61a extends in the axial direction and opens to the proximal end side. The housing hole 61a slidably accommodates an operation portion 70 (described later) of the shaft assembly 26 in the axial direction. An engagement hole 59 is formed in an upper end portion of the housing portion 61. The engagement hole 59 penetrates an upper end wall of the housing portion 61 and communicates with the housing hole 61a.

As illustrated in Fig. 4A, the support plate 54 is a plate-like member extending in a width direction from a lower end of the main body portion 53. The support plate 54 is formed in a trapezoidal shape in plan view and is provided in a pair on one side portion 52a and the other side portion 52b of the main body portion 53. The support plate 54 is formed in a flat plate shape parallel to a bottom surface 40d of the rectangular tube portion 40.

The grip ring 56 is a cylindrical member extending in the width direction from the vicinity of an upper end of the main body portion 53. The grip ring 56 is provided in pair on the one side portion 52a and the other side portion 52b of the main body portion 53. The grip ring 56 is formed in a trapezoidal shape in plan view, and a finger hook hole 60 penetrating in the vertical direction is formed therein. An outer peripheral portion of the grip ring 56 has the same shape as that of the support plate 54 in top view.

The finger hook hole 60 of the grip ring 56 is formed in a trapezoidal shape whose axial dimension becomes narrower toward the outside in the width direction. A user adjusts a width-direction position at which the finger is to be inserted into the finger hook hole 60 in accordance with a thickness of the finger, and thereby the finger can be arranged on the grip ring 56 without generating play in the axial direction, so that accurate operation can be performed.

Next, the shaft assembly 26 will be described. As illustrated in Fig. 2, the shaft assembly 26 has a shaft 62 and a shaft hub 64 joined to a proximal end of the shaft 62. Among them, the shaft 62 is formed in a columnar shape having an outer diameter that can be inserted into the lumen 28b of the outer cylinder 28. The shaft 62 is formed with, for example, a metal such as stainless steel. The shaft 62 is not limited to a cylindrical shape and may be a hollow cylindrical shape.

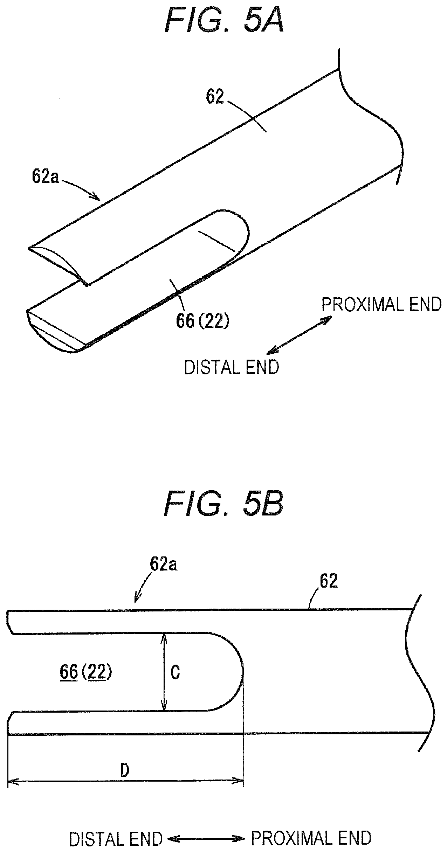

As illustrated in Figs. 5A and 5B, a second insertion hole 66 is formed at a distal end 62a of the shaft 62. The second insertion hole 66 is formed so as to penetrate a side portion of the shaft 62 in the radial direction. The second insertion hole 66 extends long in the axial direction, opens at the distal end of the shaft 62, and is formed as a U-shaped notch groove in a side view. The second insertion hole 66 is formed in a portion (corresponding portion) inside the first insertion hole 34 in the shaft 62. A width (dimension C in the circumferential direction) of the second insertion hole 66 is larger than the diameter of the implanted body 110. A length (dimension D in the axial direction) of the second insertion hole 66 is formed to be larger than the width.

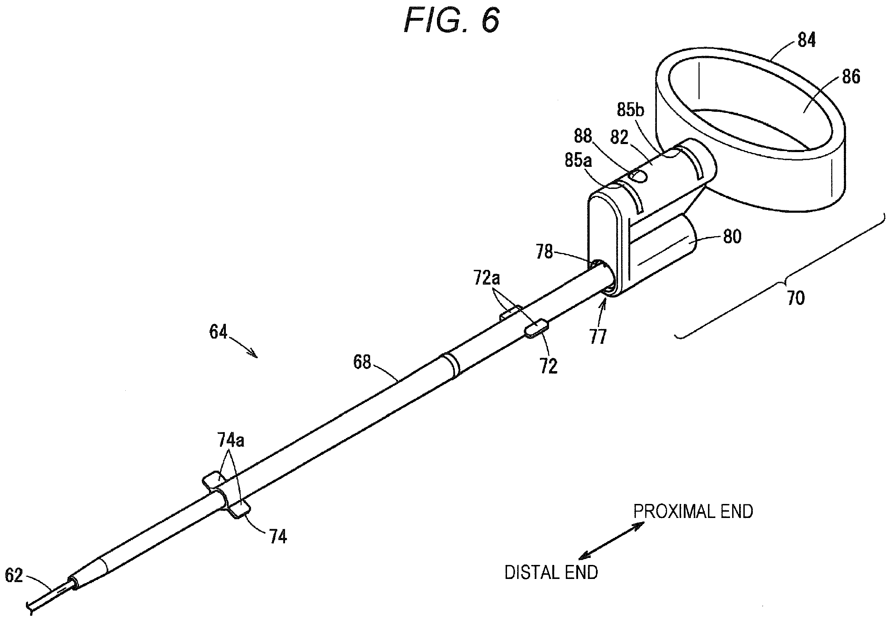

As illustrated in Fig. 6, the shaft hub 64 is provided at the proximal end of the shaft 62. The shaft hub 64 is joined to the shaft 62 and includes a cylindrical rotation shaft portion 68 elongated in the axial direction and the operation portion 70 provided on the proximal end side of the rotation shaft portion 68. The rotation shaft portion 68 is a member formed with a resin material such as a polycarbonate resin, for example, and is joined to the proximal end of the shaft 62. The rotation shaft portion 68 is joined to the shaft 62 so as to rotate integrally therewith. On an outer peripheral portion of the rotation shaft portion 68, a first blade 72 and a second blade 74 are formed so as to project outward in the radial direction.

The first blade 72 is a projection provided near the proximal end of the rotation shaft portion 68. The first blade 72 includes a pair of blade-shaped projections 72a facing each other across the center of the rotation shaft portion 68. The second blade 74 is a projection provided near the distal end of the rotation shaft portion 68 and includes a pair of blade-shaped projections 74a disposed at positions facing each other.

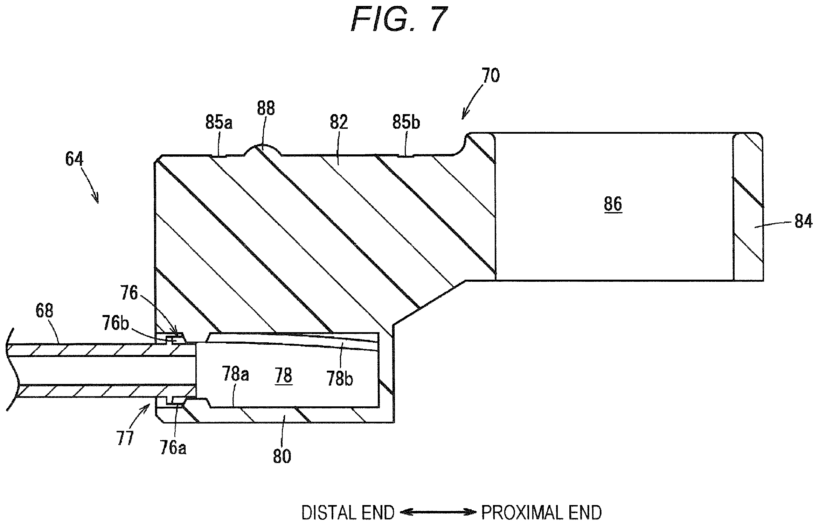

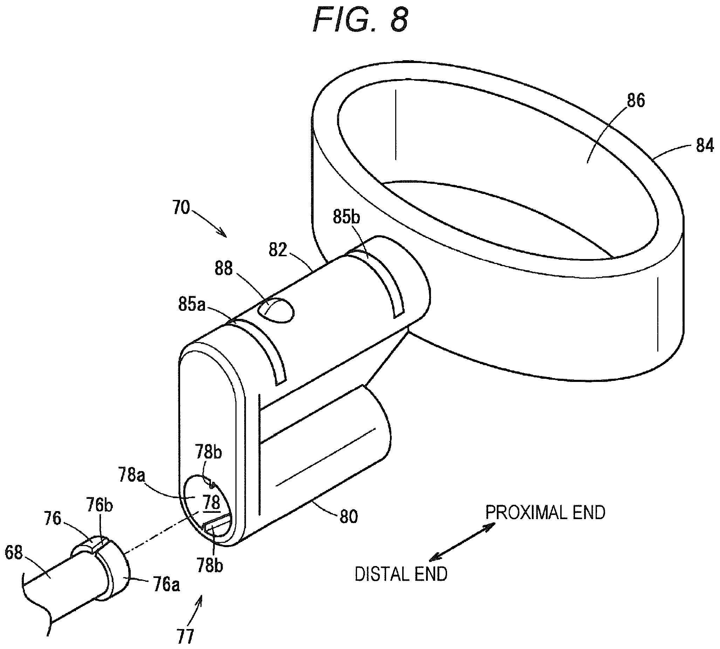

As illustrated in Fig. 8, a sliding portion 76 projecting outward in the radial direction is provided at the proximal end of the rotation shaft portion 68. The sliding portion 76 is formed with a constant projection height over the entire circumference of the rotation shaft portion 68. As illustrated in Fig. 7, an outer peripheral surface 76a of the sliding portion 76 slides while abutting on a rotation shaft portion housing hole 78 of the operation portion 70 described later. As illustrated in Fig. 8, a screw groove 76b formed by cutting out the sliding portion 76 is formed in part of the sliding portion 76 in the circumferential direction. The screw groove 76b is formed to be slightly inclined with respect to the axial direction. A pair of screw grooves 76b is provided at positions facing each other in the circumferential direction of the rotation shaft portion 68.

As illustrated in Fig. 6, the operation portion 70 includes a shaft housing portion 80 in which the rotation shaft portion housing hole 78 is formed. The shaft housing portion 80 is formed in a cylindrical shape coaxial with a central axis of the rotation shaft portion housing hole 78. A connection portion 82 is integrally connected to the shaft housing portion 80 in an upper portion of the shaft housing portion 80. An upper end of the connection portion 82 is formed in a cylindrical shape. In the operation portion 70, the shaft housing portion 80 and the connection portion 82 slide along the housing hole 61a (see Fig. 4B) of the outer cylinder hub 30.

As illustrated in Fig. 8, a distal end graduation line 85a, a proximal end graduation line 85b, and an engagement projection 88 are formed at an upper end of the connection portion 82. The distal end graduation line 85a and the proximal end graduation line 85b are groove-shaped structures extending in the width direction and are provided apart from each other in the axial direction. The distal end graduation line 85a is a line indicating that the gripping mechanism 22 is in the released state and is provided at a position coinciding with the proximal end of the main body portion 53 when the operation portion 70 is pulled out to the most proximal end side. The engagement projection 88 is provided between the distal end graduation line 85a and the proximal end graduation line 85b. The engagement projection 88 is provided at a position to be engaged with the engagement hole 59 when the operation portion 70 is pushed in, and maintains the gripping mechanism 22 in the released state. The proximal end graduation line 85b is provided at a portion coinciding with the proximal end of the housing portion 61 in a state where the engagement projection 88 is engaged with the engagement hole 59.

An operation ring 84 is formed at a proximal end of the connection portion 82. The operation ring 84 is formed in an elliptical shape in plan view. A finger hook hole 86 is formed through the operation ring 84 in the vertical direction. The finger hook hole 86 is a portion where the user mainly hooks the thumb and is formed to have a larger diameter than the finger hook hole 60 of the outer cylinder hub 30. The finger hook hole 86 is formed in an elliptical shape, so that the user can hook the finger without a gap in the axial direction by adjusting the finger in the width direction according to a size of the finger.

As illustrated in Fig. 7, the rotation shaft portion housing hole 78 inside the shaft housing portion 80 is formed to extend in the axial direction. The rotation shaft portion housing hole 78 accommodates the sliding portion 76 at the proximal end of the rotation shaft portion 68 so as to be slidable in the axial direction. As illustrated in Figs. 7 and 8, a pair of screw threads 78b is provided on an inner peripheral surface 78a of the rotation shaft portion housing hole 78 so as to project inward. The screw thread 78b is provided at a portion to be engaged with the screw groove 76b and extends while rotating in the axial direction.

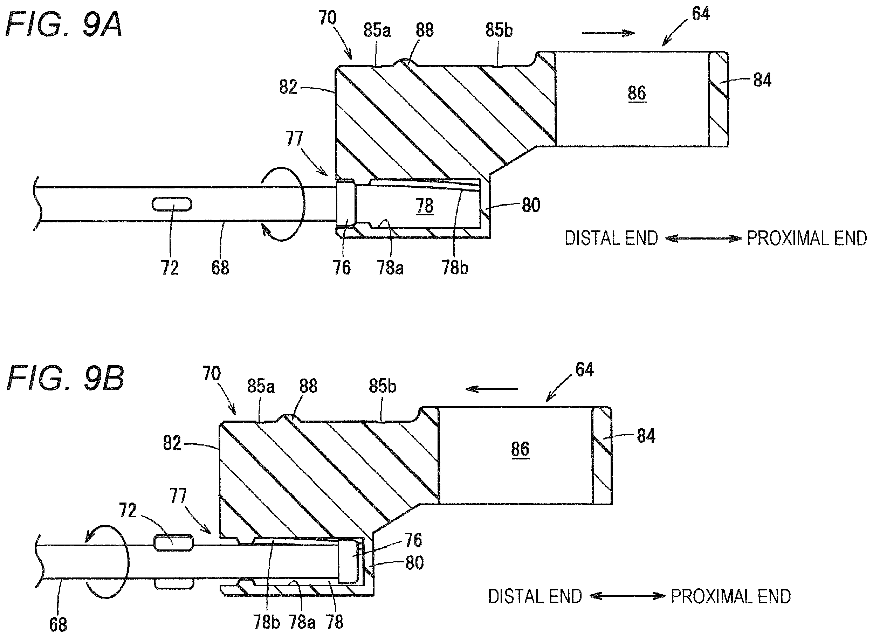

As illustrated in Figs. 9A and 9B, when the screw groove 76b slides in the axial direction while being engaged with the screw thread 78b, rotational displacement is generated in the rotation shaft portion 68. In other words, a rotation mechanism 77 of the present embodiment is configured by a screw structure having the screw thread 78b and the screw groove 76b. As illustrated in Fig. 9A, when the user performs operation of pulling out the operation portion 70 from the rotation shaft portion 68, counterclockwise rotational motion is generated as viewed from the proximal end side (user side). Further, as illustrated in Fig. 9B, when the user performs operation of pushing the operation portion 70 toward the rotation shaft portion 68, clockwise rotational motion is generated as viewed from the proximal end side. Note that the rotation direction is not limited to the above example and may be the opposite direction.

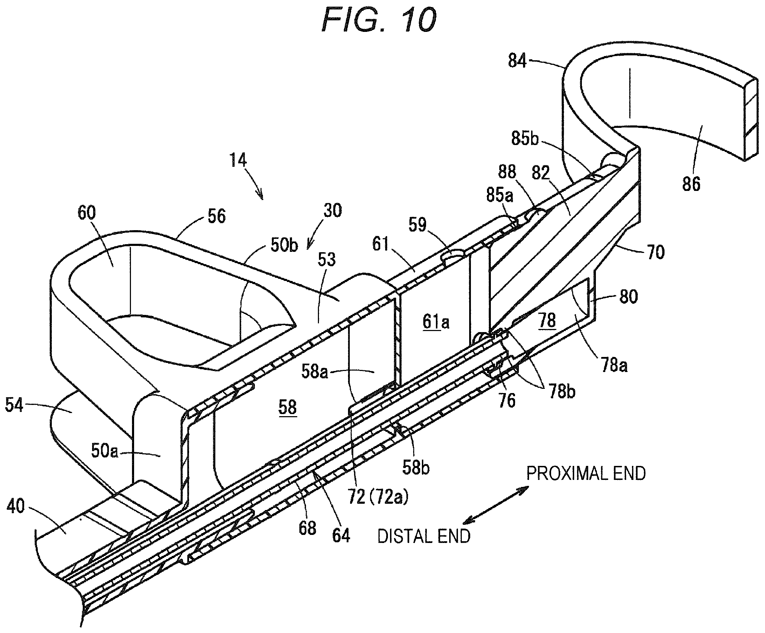

As illustrated in Fig. 10, the shaft hub 64 is mounted inside the outer cylinder hub 30. The rotation shaft portion 68 of the shaft hub 64 is accommodated in the shaft hole 58b of the outer cylinder hub 30, the first cavity 46, and the second cavity 58 through the shaft hole 38b of Fig. 4B. The operation portion 70 of the shaft hub 64 is accommodated in the housing portion 61 of the grip portion 50 of the outer cylinder hub 30 so as to be slidable in the axial direction. The operation portion 70 and the housing hole 61a of the housing portion 61 are formed in a non-circular shape, and thus, the operation portion 70 is displaced in the axial direction without rotating. On the other hand, the rotation shaft portion 68 is rotatably accommodated with respect to the outer cylinder hub 30.

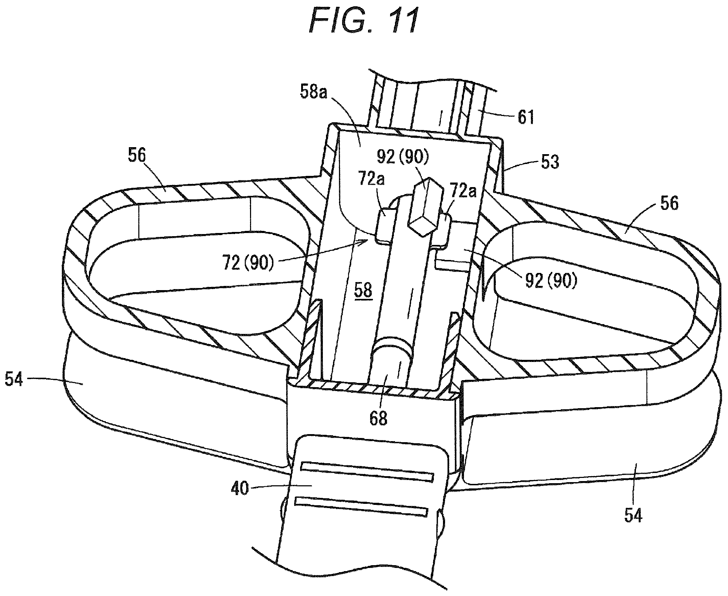

As illustrated in Fig. 11, a pair of rotation restricting projections 92 as a rotation restricting portion 90 is formed on the proximal end wall 58a of the outer cylinder hub 30. The pair of rotation restricting projections 92 is provided at a predetermined angle in the circumferential direction of the rotation shaft portion 68, and the first blade 72 is disposed between the pair of rotation restricting projections 92. The rotation restricting projection 92 comes into contact with the first blade 72 to restrict a rotation angle range of the rotation shaft portion 68 to a range of the released state and a range of the fixed state. The rotation restricting portion 90 includes the first blade 72 and the rotation restricting projection 92. The first blade 72 abuts on the proximal end wall 58a to prevent movement of the rotation shaft portion 68 toward the proximal end side.

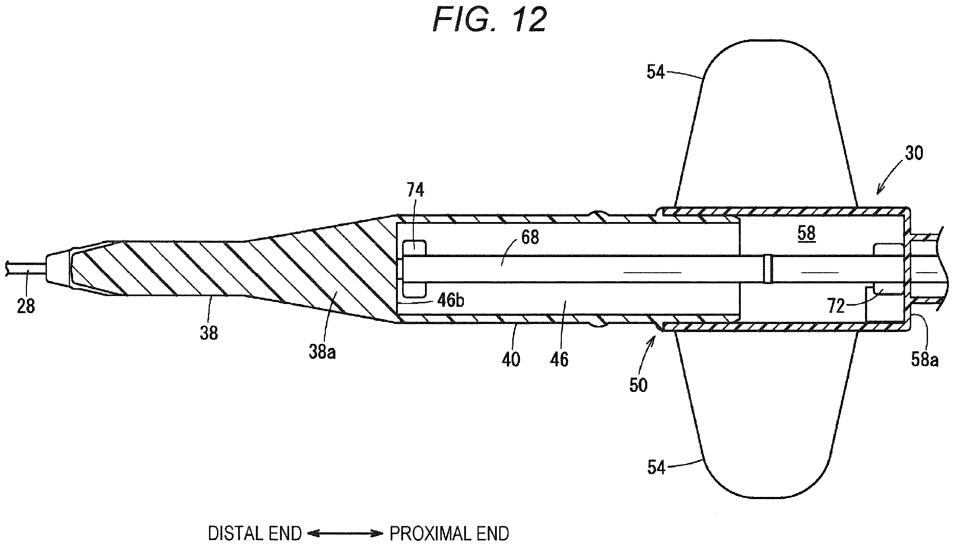

As illustrated in Fig. 12, the second blade 74 abuts on the distal end wall 46b of the outer cylinder hub 30 from the proximal end side to prevent movement of the rotation shaft portion 68 toward the distal end side. In other words, the distal end wall 46b and the proximal end wall 58a, and the first blade 72 and the second blade 74 prevent axial displacement of the rotation shaft portion 68 with respect to the outer cylinder hub 30.

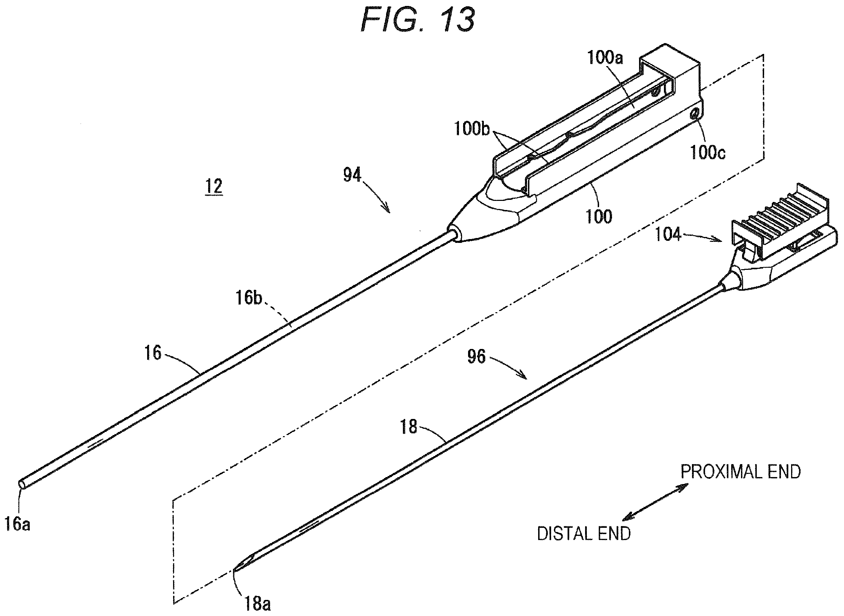

Next, the puncture needle 12 of the implanted body indwelling instrument 10 will be described. As illustrated in Fig. 13, the puncture needle 12 includes the outer needle assembly 94 and the inner needle assembly 96. The inner needle assembly 96 is detachably attached to the outer needle assembly 94 from the proximal end side of the outer needle assembly 94. The outer needle assembly 94 includes a cylindrical outer needle 16 having a blunt distal end 16a and an outer needle hub 100 provided at a proximal end of the outer needle 16. A lumen 16b extending in the axial direction is formed to penetrate the outer needle 16. A cavity 100a is formed in the outer needle hub 100. The cavity 100a opens in a guide groove 100b at an upper end. In addition, engagement holes 100c are formed in both side portions on the proximal end side of the outer needle hub 100.

The inner needle assembly 96 includes an inner needle 18 having a sharp distal end 18a and an inner needle hub 104 provided on the proximal end side of the inner needle 18. The inner needle 18 is disposed slidably in the axial direction in the lumen 16b of the outer needle 16. When the inner needle hub 104 is pushed forward in the axial direction, the sharp distal end 18a of the inner needle 18 projects from the distal end 16a of the outer needle 16. The inner needle assembly 96 can be pulled out from the proximal end side of the outer needle assembly 94. The puncture needle 12 is used by puncturing from the skin 112 of the patient (living body) to a target site in a living tissue in a state where the inner needle assembly 96 is attached to the outer needle assembly 94.

The implanted body indwelling instrument 10 and the operation device 14 of the present embodiment are configured as described above, and the operation thereof will be described below together with the method of use.

First, operation of attaching the implanted body 110 to the operation device 14 will be described. The user inserts the index finger and the middle finger into the finger hook hole 60 of the outer cylinder hub 30 of the operation device 14 of Fig. 1 and inserts the thumb into the finger hook hole 86 of the shaft hub 64 to grip the operation device 14. In the operation device 14 in the initial state, as illustrated in Fig. 14B, the operation portion 70 of the shaft hub 64 is pulled out to the proximal end side, and the engagement projection 88 is located on the proximal end side of the outer cylinder hub 30. In this event, the distal end graduation line 85a overlaps the proximal end of the housing portion 61 of the outer cylinder hub 30. In this state, as illustrated in Fig. 14A, the second insertion hole 66 of the shaft 62 and the first insertion hole 34 of the outer cylinder 28 are in a released state in which positions in the circumferential direction coincide with each other. The user can visually recognize the released state from the position of the distal end graduation line 85a illustrated in Fig. 8.

Next, as illustrated in Fig. 14A, the user grips the thread-like implanted body 110 with tweezers, or the like, and disposes the implanted body 110 so as to pass through the first insertion hole 34 and the second insertion hole 66.

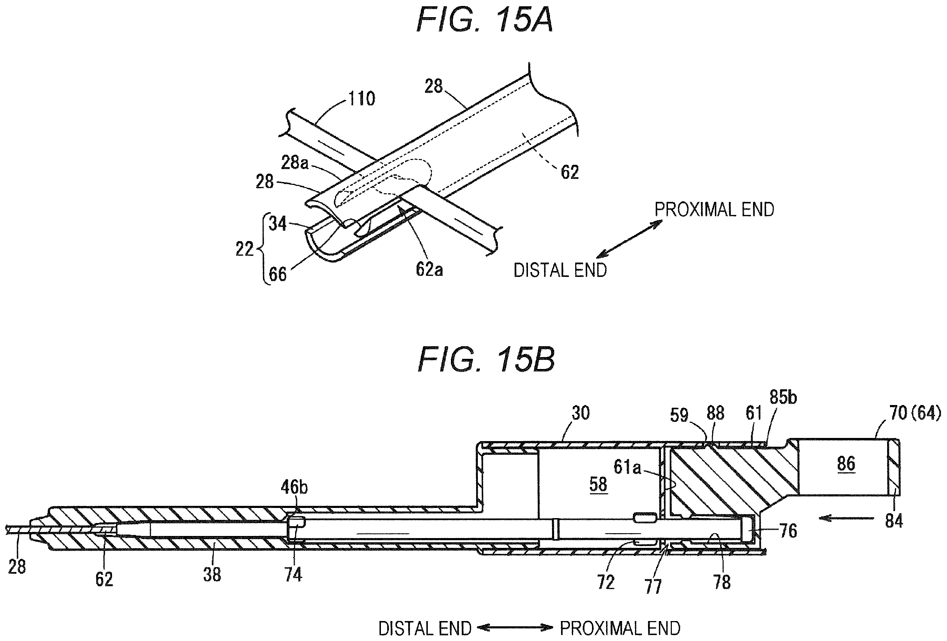

Next, as illustrated in Fig. 15B, the user performs operation of pushing the operation portion 70 toward the outer cylinder hub 30. By this operation, the engagement projection 88 of the operation portion 70 is engaged with the engagement hole 59 of the outer cylinder hub 30 and stopped. When the operation portion 70 is displaced toward the distal end side, the sliding portion 76 of the rotation shaft portion 68 slides in the axial direction inside the rotation shaft portion housing hole 78. Then, the axial displacement of the operation portion 70 is converted into the rotational displacement of the rotation shaft portion 68 (see Fig. 7) by the screw groove 76b and the screw thread 78b constituting the rotation mechanism 77 illustrated in Fig. 7, and the rotation shaft portion 68 rotates.

As the rotation shaft portion 68 rotates, as illustrated in Fig. 15A, the first insertion hole 34 and the second insertion hole 66 are displaced in the circumferential direction, the implanted body 110 is sandwiched and fixed between the shaft 62 and the outer cylinder 28, and the implanted body 110 is in an undetachable fixed state. As illustrated in Fig. 15B, the operation device 14 is maintained in the fixed state by the engagement of the engagement projection 88 of the operation portion 70 and the engagement hole 59 of the outer cylinder hub 30. In this state, the positions of the proximal end graduation line 85b and the proximal end of the housing portion 61 coincide with each other. Through the above operation, attachment of the implanted body 110 to the operation device 14 is completed.

Next, as illustrated in Fig. 16, the user punctures a target site of the patient (living body) with the puncture needle 12. After puncturing with the puncture needle 12, the user pulls out the inner needle assembly 96 from the outer needle assembly 94 while leaving the outer needle assembly 94 of the puncture needle 12.

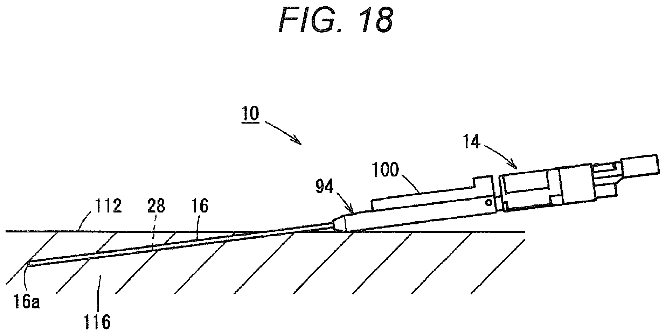

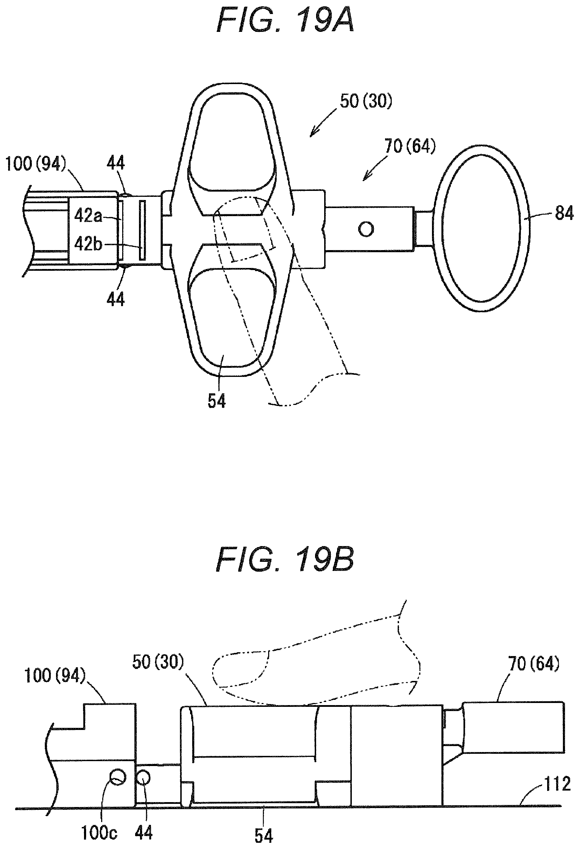

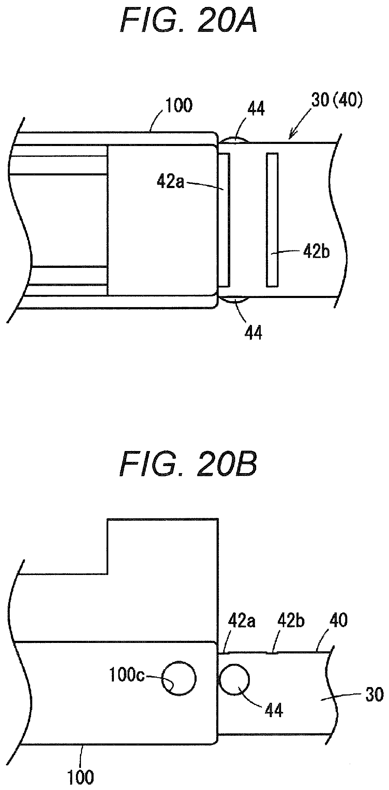

Next, as illustrated in Figs. 17 and 18, the user inserts the operation device 14 holding the implanted body 110 from the proximal end of the outer needle hub 100 of the outer needle assembly 94. Then, the user inserts the outer cylinder 28 of the operation device 14 into the lumen 16b of the outer needle 16 of the outer needle assembly 94 and moves the implanted body 110 into the body of the living body through the inside of the lumen 16b. The implanted body 110 is protected by the outer needle 16, and thus, it does not receive resistance when advancing in the living body. As a result, even if the target site is a deep portion of the living tissue 116, the implanted body 110 can be conveyed without being broken. As illustrated in Figs. 19A and 19B, when the outer cylinder hub 30 of the operation device 14 is made to advance, the lock projection 44 abuts on the proximal end of the outer needle hub 100, whereby the operation device 14 stops. In this state, the gripping mechanism 22 of the operation device 14 is accommodated inside the outer needle 16. As illustrated in Figs. 20A and 20B, the first graduation line 42a of the outer cylinder hub 30 overlaps the proximal end of the outer needle hub 100, so that the user can visually recognize the position of the distal end 14a of the operation device 14.

Next, the user performs operation to cause the gripping mechanism 22 of the operation device 14 to project beyond the distal end 16a of the outer needle 16. Here, the operation device 14 is fixed to the living tissue 116, and the outer needle assembly 94 is moved to the proximal end side in order to prevent displacement of the indwelling position of the implanted body 110 and to prevent action of a frictional load on the implanted body 110. In other words, as illustrated in Figs. 19A and 19B, in order to fix the operation device 14 to the living tissue 116, the user presses the grip portion 50 of the outer cylinder hub 30 against the skin 112 with the finger. Thus, the support plate 54 of the grip portion 50 comes into surface contact with the skin 112, and the grip portion 50 is securely fixed in a stable state.

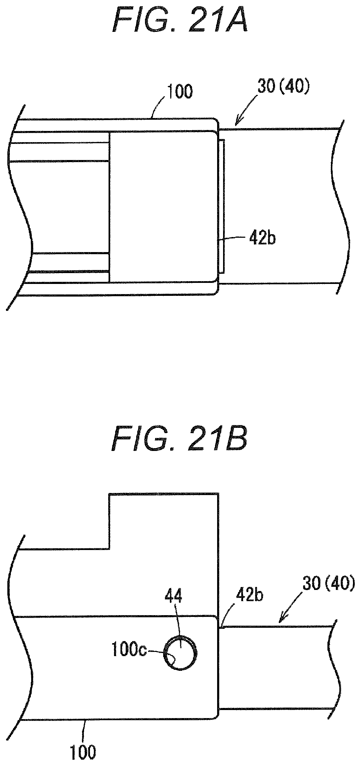

Thereafter, the user grips the outer needle hub 100 and slides the outer needle hub 100 toward the outer cylinder hub 30 to the proximal end side. As a result, as illustrated in Figs. 21A and 21B, the lock projection 44 is engaged with the engagement hole 100c of the outer needle hub 100. The lock projection 44 fixes the gripping mechanism 22 of the operation device 14 in a state where the gripping mechanism 22 projects from the distal end 16a of the outer needle 16. In this state, as illustrated in Fig. 21A, the second graduation line 42b coincides with the proximal end of the outer needle hub 100, so that the user can visually recognize the projection of the gripping mechanism 22 at the position of the second graduation line 42b.

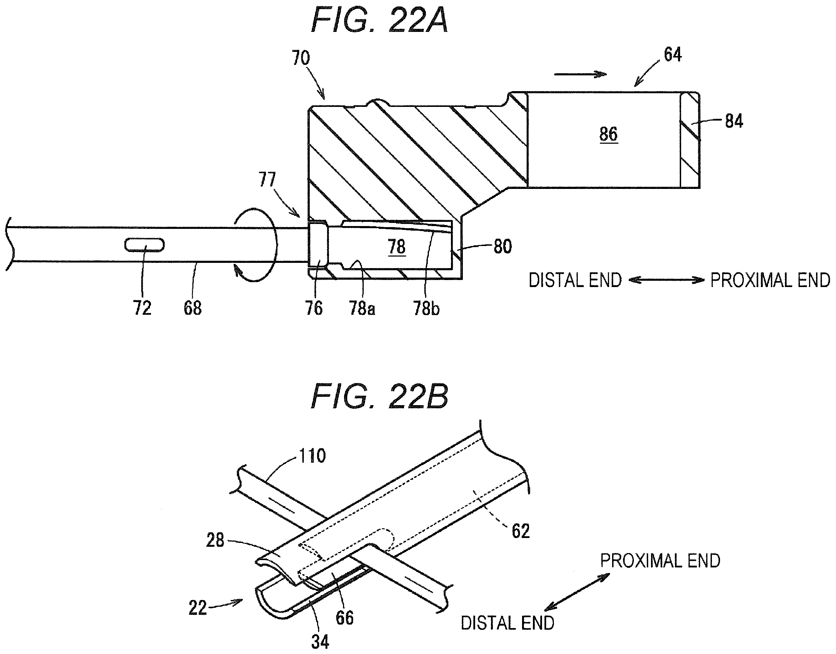

Next, in order to switch the fixed state of the gripping mechanism 22 of the operation device 14 to the released state, the user performs operation of pulling out the operation portion 70 to the proximal end side as illustrated in Fig. 22A. When the operation portion 70 is pulled out toward the proximal end side, the shaft 62 rotates together with the rotation shaft portion 68, and the positions in the circumferential direction of the first insertion hole 34 of the outer cylinder 28 and the second insertion hole 66 of the shaft 62 coincide with each other as illustrated in Fig. 22B. As a result, the gripping mechanism 22 of the operation device 14 is in a released state in which the implanted body 110 can be detached.

The gripping mechanism 22 of the operation device 14 of the present embodiment can perform operation of switching between the fixed state and the released state only by rotating the shaft 62 inside the outer cylinder 28 without projecting outward from the outer cylinder 28. Thus, in the gripping mechanism 22 of the present embodiment, even when a pressure in the living tissue 116 acts, releasing operation is less likely to be hindered, and the implanted body 110 can be reliably released even in the deep portion of the living tissue 116.

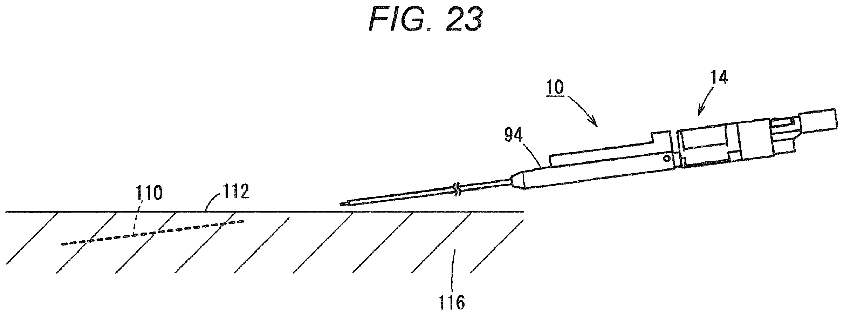

Thereafter, as illustrated in Fig. 23, the user pulls out the operation device 14 from the skin 112 of the living body together with the outer needle assembly 94. The implanted body 110 is separated from the gripping mechanism 22 by friction with the living tissue 116 and indwelled in the living tissue 116.

The operation device 14 and the implanted body indwelling instrument 10 of the present embodiment have the following effects.

The operation device 14 of the present embodiment includes the outer cylinder 28 in which the lumen 28b penetrating in an axial direction is formed, the shaft 62 disposed in the lumen 28b, and the gripping mechanism 22 that is provided in the outer cylinder 28 and the shaft 62 and grips the thread-like implanted body 110 in a switchable manner between an undetachable fixed state and a detachable released state. The gripping mechanism 22 includes the first insertion hole 34 that penetrates a side portion of the outer cylinder 28 in the radial direction, the second insertion hole 66 that is provided in the shaft 62 at a portion corresponding to the first insertion hole 34 and penetrates a side portion of the shaft 62 in the radial direction, and the rotation mechanism 77 that rotates the shaft 62 relative to the outer cylinder 28. The implanted body 110 is inserted into the first insertion hole 34 and the second insertion hole 66 and gripped.

According to the operation device 14 described above, it is possible to perform operation of releasing the implanted body 110 only by rotating the shaft 62 inside the outer cylinder 28 without projecting outward from the outer cylinder 28. Thus, the implanted body 110 can be reliably released even inside the living tissue 116 on which a pressure acts, so that the implanted body 110 can be reliably indwelled inside the living tissue 116.

In the operation device 14 described above, the first insertion hole 34 and the second insertion hole 66 may be opened at the distal end and formed in a groove shape. According to this configuration, the side portion of the implanted body 110 can be disposed in the first insertion hole 34 and the second insertion hole 66 by sliding the side portion from the distal end side to the proximal end side. It is not necessary to pass the distal end of the soft implanted body 110 through the first insertion hole 34 and the second insertion hole 66, which makes attachment of the implanted body 110 easy.

In the operation device 14 described above, circumferential dimensions of the first insertion hole 34 and the second insertion hole 66 may be larger than the diameter of the implanted body 110, and axial dimensions of the first insertion hole 34 and the second insertion hole 66 may be larger than the circumferential dimensions. According to this configuration, the implanted body 110 can be easily attached to the first insertion hole 34 and the second insertion hole 66. In addition, the first insertion hole 34 and the second insertion hole 66 extend long in the axial direction, so that even if the positions of the outer cylinder 28 and the shaft 62 in the axial direction slightly deviate, the operation is not hindered.

In the operation device 14 described above, in the fixed state, the positions of the first insertion hole 34 and the second insertion hole 66 in the circumferential direction are shifted, and the implanted body 110 is sandwiched between the shaft 62 and the outer cylinder 28. According to this configuration, the implanted body 110 can be reliably fixed in the gripping mechanism 22.

In the operation device 14 described above, the positions of the first insertion hole 34 and the second insertion hole 66 in the circumferential direction coincide with each other in the released state. According to this configuration, the implanted body 110 can be released without performing the operation of projecting outward from the outer cylinder 28, so that the releasing operation can be reliably performed even inside the living tissue 116.

The operation device 14 may further include the outer cylinder hub 30 joined to the proximal end of the outer cylinder 28, and the shaft hub 64 that supports the proximal end of the shaft 62 and is attached to the outer cylinder hub 30 so as to be movable in the axial direction. The shaft hub 64 may include the rotation shaft portion 68 that is joined to the proximal end of the shaft 62 and rotates integrally with the shaft 62, and the operation portion 70 having the rotation shaft portion housing hole 78 that slidably accommodates the rotation shaft portion 68 in the axial direction. The rotation mechanism 77 may include a screw mechanism that is formed by an inner wall of the rotation shaft portion housing hole 78 and an outer periphery of the rotation shaft portion 68 and converts axial displacement of the operation portion 70 into rotational displacement of the rotation shaft portion 68.

According to the above configuration, the rotational displacement can be generated in the shaft 62 by the operation of the shaft hub 64, so that excellent operability can be achieved.

The operation device 14 may further include the rotation restricting portion 90 that restricts the rotation range of the rotation shaft portion 68. According to this configuration, the rotation range of the rotation shaft portion 68 can be set to the range between the released state and the fixed state, and even when the gripping mechanism 22 is inserted into the living body and cannot be visually recognized, reliable operation can be performed.

In the operation device 14 described above, the rotation restricting portion 90 may include the rotation restricting projection 92 provided on the outer cylinder hub 30 and the first blade 72 projecting outward from the rotation shaft portion 68. According to this configuration, rotation can be restricted with a simple device configuration.

In the operation device 14 described above, the rotation shaft portion 68 may include the second blade 74 projecting outward, and the outer cylinder hub 30 may have a wall (distal end wall 46b) that abuts on the second blade 74 from the axial direction to prevent the axial displacement of the rotation shaft portion 68 with respect to the outer cylinder hub 30.

The implanted body indwelling instrument 10 of the present embodiment includes the operation device 14 described above, and the puncture needle 12 having the outer needle 16 into which the outer cylinder 28 of the operation device 14 can be inserted in a state where the implanted body 110 is gripped. According to the implanted body indwelling instrument 10, the implanted body 110 can be reliably indwelled in the living tissue 116.

Although understanding of the invention has been provided by reference to the preferred embodiments, the present invention is not limited to the above-described embodiments, and it goes without saying that various modifications can be made without departing from the scope of the invention which is defined by the following claims

## Claims

1. An implanted body indwelling instrument (10) comprising:
an operation device (14); comprising:
an outer cylinder (28) in which a lumen (28b) penetrating in an axial direction is formed;
a shaft (62) disposed in the lumen (28b); and
a gripping mechanism (22) that is provided in the outer cylinder (28) and the shaft (62) and grips a thread-like implanted body (110) in a switchable manner between a fixed state in which the thread-like implanted body (110) is undetachable and a released state in which the thread-like implanted body (110) is detachable,
wherein the gripping mechanism (22) includes:
a first insertion hole (34) penetrating a side portion of the outer cylinder (28) in a radial direction;
a second insertion hole (66) provided in the shaft (62) at a portion corresponding to the first insertion hole (34) and penetrating a side portion of the shaft (62) in the radial direction; and
a rotation mechanism (77) that rotates the shaft (62) relative to the outer cylinder (28), and
the implanted body (110) is inserted into the first insertion hole (34) and the second insertion hole (66) and gripped,
and
a puncture needle (12) having an outer needle (16) into which the outer cylinder (28) of the operation device (14) is insertable in a state where the implanted body (110) is gripped by the gripping mechanism (22), wherein the operation device (14) is configured to convey the implanted body (110) through the outer needle (16) from its distal side to a target site at the proximal end of the puncture needle (12).

2. The implanted body indwelling instrument (10) according to claim 1, wherein the first insertion hole (34) and the second insertion hole (66) are formed in a groove shape opened at a distal end.

3. The implanted body indwelling instrument (10) according to claim 1 or 2, wherein circumferential dimensions of the first insertion hole (34) and the second insertion hole (66) are larger than a diameter of the implanted body (110), and
axial dimensions of the first insertion hole (34) and the second insertion hole (66) are larger than the circumferential dimensions.

4. The implanted body indwelling instrument (10) according to any one of claims 1 to 3, wherein in the fixed state, positions of the first insertion hole (34) and the second insertion hole (66) in the circumferential direction are shifted, and the implanted body (110) is sandwiched between the shaft (62) and the outer cylinder (28).

5. The implanted body indwelling instrument (10) according to any one of claims 1 to 4, wherein in the released state, positions of the first insertion hole (34) and the second insertion hole (66) in the circumferential direction coincide with each other.

6. The implanted body indwelling instrument (10) according to any one of claims 1 to 5, further comprising:
an outer cylinder hub (30) joined to a proximal end of the outer cylinder (28); and
a shaft hub (64) that supports a proximal end of the shaft (62) and is attached to the outer cylinder hub (30) so as to be movable in an axial direction,
wherein the shaft hub (64) includes a rotation shaft portion (68) that is joined to the proximal end of the shaft (62) and rotates integrally with the shaft (62), and an operation portion (70) having a rotation shaft portion housing hole (78) that slidably accommodates the rotation shaft portion (68) in the axial direction, and
the rotation mechanism (77) includes a screw mechanism that is formed by an inner wall of the rotation shaft portion housing hole (78) and an outer periphery of the rotation shaft portion (68) and converts axial displacement of the operation portion (70) into rotational displacement of the rotation shaft portion (68).

7. The implanted body indwelling instrument (10) according to claim 6, further comprising a rotation restricting portion (90) that restricts a rotation range of the rotation shaft portion (68).

8. The implanted body indwelling instrument (10) according to claim 7, wherein the rotation restricting portion (90) includes a rotation restricting projection (92) provided on the outer cylinder hub (30) and a first blade (72) projecting outward from the rotation shaft portion (68).

9. The implanted body indwelling instrument (10) according to any one of claims 6 to 8, wherein the rotation shaft portion (68) includes a second blade (74) projecting outward, and the outer cylinder hub (30) has a wall (46b) that abuts on the second blade (74) from the axial direction to prevent axial displacement of the rotation shaft portion (68) with respect to the outer cylinder hub (30).

## Patentansprüche

1. Implantiertes Körper-Verweilinstrument (10), umfassend:
eine Betätigungsvorrichtung (14), umfassend:
einen Außenzylinder (28), in dem ein Lumen (28b) ausgebildet ist, das in einer axialen Richtung verläuft;
einen Schaft (62), der in dem Lumen (28b) angeordnet ist; und
einen Greifmechanismus (22), der in dem Außenzylinder (28) und dem Schaft (62) vorgesehen ist und ein fadenförmiger implantierter Körper (110) in einer umschaltbaren Weise zwischen einem fixierten Zustand, in welchem der fadenförmige implantierte Körper (110) nicht lösbar ist, und einem freigegebenen Zustand, in dem der fadenförmige implantierte Körper (110) lösbar ist,
wobei der Greifmechanismus (22) umfasst:
eine erste Einführungsöffnung (34), die einen Seitenabschnitt des Außenzylinders (28) in einer radialen Richtung durchdringt;
eine zweite Einführungsöffnung (66), die in dem Schaft (62) an einem Abschnitt vorgesehen ist, welcher der ersten Einführungsöffnung (34) entspricht, und einen Seitenabschnitt des Schafts (62) in der radialen Richtung durchdringt; und
einen Rotationsmechanismus (77), der den Schaft (62) relativ zu dem Außenzylinder (28) dreht, und
der implantierte Körper (110) in die erste Einführungsöffnung (34) und die zweite Einführungsöffnung (66) eingeführt und gegriffen wird,
und
eine Punktionsnadel (12), die eine äußere Nadel (16) aufweist, in welche der Außenzylinder (28) der Betätigungsvorrichtung (14) in einem Zustand eingeführt werden kann, in welchem der implantierte Körper (110) durch den Greifmechanismus (22) gegriffen wird, wobei die Betätigungsvorrichtung (14) so konfiguriert ist, dass sie den implantierten Körper (110) durch die äußere Nadel (16) von ihrer distalen Seite zu einer Zielstelle an dem proximalen Ende der Punktionsnadel (12) transportiert.

2. Implantiertes Körper-Verweilinstrument (10) nach Anspruch 1, wobei die erste Einführungsöffnung (34) und die zweite Einführungsöffnung (66) in einer Nutform ausgebildet sind, welche an dem distalen Ende offen ist.

3. Implantiertes Körper-Verweilinstrument (10) nach Anspruch 1 oder 2, wobei die Umfangsabmessungen von der ersten Einführungsöffnung (34) und der zweiten Einführungsöffnung (66) größer sind als der Durchmesser des implantierten Körpers (110), und
die axialen Abmessungen der ersten Einführungsöffnung (34) und der zweiten Einführungsöffnung (66) größer sind als die Umfangsabmessungen.

4. Implantiertes Körper-Verweilinstrument (10) nach einem der Ansprüche 1 bis 3, wobei in dem fixierten Zustand die Positionen der ersten Einführungsöffnung (34) und der zweiten Einführungsöffnung (66) in Umfangsrichtung verschoben sind und der implantierte Körper (110) zwischen dem Schaft (62) und dem Außenzylinder (28) sandwichartig eingeschlossen ist.

5. Implantiertes Körper-Verweilinstrument (10) nach einem der Ansprüche 1 bis 4, wobei in dem freigegebenen Zustand die Positionen der ersten Einführungsöffnung (34) und der zweiten Einführungsöffnung (66) in der Umfangsrichtung miteinander übereinstimmen.

6. Implantiertes Körper-Verweilinstrument (10) nach einem der Ansprüche 1 bis 5, ferner umfassend:
eine Außenzylindernabe (30), die mit einem proximalen Ende des Außenzylinders (28) verbunden ist; und
eine Schaftnabe (64), die ein proximales Ende des Schafts (62) stützt und an der Außenzylindernabe (30) angebracht ist, sodass sie in einer axialen Richtung beweglich ist,
wobei die Schaftnabe (64) einen Drehschaftabschnitt (68) umfasst, der mit dem proximalen Ende des Schafts (62) verbunden ist und sich integral mit dem Schaft (62) dreht, und einen Betätigungsabschnitt (70), der eine Drehschaftabschnitt-Gehäuseöffnung (78) aufweist, die den Drehschaftabschnitt (68) in der axialen Richtung verschiebbar aufnimmt, und
der Rotationsmechanismus (77) einen Schraubmechanismus umfasst, der durch eine Innenwand der Drehschaftabschnitt-Gehäuseöffnung (78) und einem Außenumfang des Drehschaftabschnitts (68) ausgebildet wird und eine axiale Verschiebung des Betätigungsabschnitts (70) in eine Drehverschiebung des Drehschaftabschnitts (68) umwandelt.

7. Implantiertes Körper-Verweilinstrument (10) nach Anspruch 6, das ferner einen Drehbegrenzungsabschnitt (90) umfasst, der einen Drehbereich des Drehschaftabschnitts (68) begrenzt.

8. Implantiertes Körper-Verweilinstrument (10) nach Anspruch 7, wobei der Drehbegrenzungsabschnitt (90) einen Drehbegrenzungsvorsprung (92), der an der Außenzylindernabe (30) vorgesehen ist, und einen ersten Flügel (72) umfasst, der von dem Drehschaftabschnitt (68) nach außen vorsteht.

9. Implantiertes Körper-Verweilinstrument (10) nach einem der Ansprüche 6 bis 8, wobei der Drehschaftabschnitt (68) einen zweiten Flügel (74) umfasst, der nach außen vorsteht, und die Außenzylindernabe (30) eine Wand (46b) aufweist, die in der axialen Richtung an dem zweiten Flügel (74) anliegt, um eine axiale Verschiebung des Drehschaftabschnitts (68) in Bezug auf die Außenzylindernabe (30) zu verhindern.

## Revendications

1. Instrument implanté à demeure pour le corps (10) comprenant :
un dispositif de commande (14) ; comprenant :
un cylindre externe (28) dans lequel est formé un lumen (28b) pénétrant dans une direction axiale ;
un arbre (62) disposé à décharger une dans le lumen (28b) ; et
un mécanisme de préhension (22) qui est prévu dans le cylindre externe (28) et dans l'journée en gérant chez des gens de garder le Frans intérieur des suivants (62) et saisit un corps implanté (110) de type fileté de manière commutable entre un état fixe dans lequel le corps implanté filiforme (110) est indétachable et un état relâché dans lequel le corps implanté filiforme (110) est détachable,
le mécanisme de préhension (22) comprenant :
un premier trou d'insertion (34) pénétrant dans une partie latérale du cylindre externe (28) dans une direction radiale ;
un second trou d'insertion (66) ménagé dans l'arbre (62) au niveau d'une partie correspondant au premier trou d'insertion (34) et pénétrant dans une partie latérale de l'arbre (62) dans la direction radiale ; et
un mécanisme de rotation (77) faisant tourner l'arbre (62) par rapport au cylindre externe (28), et
le corps implanté (110) étant inséré dans le premier trou d'insertion (34) et le second trou d'insertion (66) et saisi, et
une aiguille de ponction (12) comportant une aiguille externe (16) dans laquelle le cylindre externe (28) du dispositif d'opération (14) peut être inséré dans un état où le corps implanté (110) est saisi par le mécanisme de préhension (22), le dispositif d'actionnement (14) étant configuré pour transporter le corps implanté (110) à travers l'aiguille externe (16) depuis son côté distal jusqu'à un site de destination à l'extrémité proximale de l'aiguille de ponction (12).

2. Instrument implanté à demeure pour le corps (10) selon la revendication 1, dans lequel le premier trou d'insertion (34) et le second trou d'insertion (66) sont formés en une forme de rainure ouverte à une extrémité distale.

3. Instrument implanté à demeure pour le corps (10) selon la revendication 1 ou 2, dans lequel les dimensions circonférentielles du premier trou d'insertion (34) et du second trou d'insertion (66) sont supérieures à un diamètre du corps implanté (110), et
les dimensions axiales du premier trou d'insertion (34) et du second trou d'insertion (66) sont supérieures aux dimensions circonférentielles.

4. Instrument implanté à demeure pour le corps (10) selon l'une quelconque des revendications 1 à 3, dans lequel, dans l'état fixe, les positions du premier trou d'insertion (34) et du second trou d'insertion (66) dans la direction circonférentielle sont décalées, et le corps implanté (110) est pris en sandwich entre l'arbre (62) et le cylindre externe (28).

5. Instrument implanté à demeure pour le corps (10) selon l'une quelconque des revendications 1 à 4, dans lequel, dans l'état relâché, les positions du premier trou d'insertion (34) et du second trou d'insertion (66) dans la direction circonférentielle coïncident l'une avec l'autre.

6. Instrument implanté à demeure pour le corps (10) selon l'une quelconque des revendications 1 à 5, comprenant en outre :
un moyeu cylindrique externe (30) relié à une extrémité proximale du cylindre externe (28) ; et
un moyeu d'arbre (64) qui supporte une extrémité proximale de l'arbre (62) et qui est fixé au moyeu cylindrique externe (30) de manière à pouvoir se déplacer dans une direction axiale,
le moyeu d'arbre (64) comprenant une partie arbre de rotation (68) qui est jointe à l'extrémité proximale de l'arbre (62) et tourne intégralement avec l'arbre (62), et une partie de fonctionnement (70) ayant un trou de logement de partie arbre de rotation (78) qui reçoit de manière coulissante la partie arbre de rotation (68) dans la direction axiale, et
le mécanisme de rotation (77) comprenant un mécanisme à vis qui est formé par une paroi interne du trou de logement de la partie arbre de rotation (78) et une périphérie externe de la partie arbre de rotation (68) et convertit le déplacement axial de la partie actionnement (70) en déplacement rotatif de la partie arbre de rotation (68).

7. Instrument implanté à demeure pour le corps (10) selon la revendication 6, comprenant en outre une partie de restriction de rotation (90) qui restreint une plage de rotation de la partie arbre de rotation (68).

8. Instrument implanté à demeure pour le corps (10) selon la revendication 7, dans lequel la partie de restriction de rotation (90) comprend une saillie de restriction de rotation (92) prévue sur le moyeu cylindrique externe (30) et une première lame (72) faisant saillie vers l'extérieur à partir de la partie arbre de rotation (68).

9. Instrument implanté à demeure pour le corps (10) selon l'une quelconque des revendications 6 à 8, dans lequel la partie arbre de rotation (68) comprend une seconde lame (74) faisant saillie vers l'extérieur, et le moyeu cylindrique externe (30) a une paroi (46b) qui bute sur la seconde lame (74) à partir de la direction axiale pour empêcher un déplacement axial de la partie arbre de rotation (68) par rapport au moyeu cylindrique externe (30).
